# EUROPÄISCHE PATENTSCHRIFT

(11) **EP 1 675 486 B1**
(45) Veröffentlichungstag und Bekanntmachung des Hinweises auf die Patenterteilung: **29.04.2015**
(21) Anmeldenummer: 04789546.1
(22) Anmeldetag: 15.10.2004
(51) Int. Cl.: A41D 19/00, A61B 19/04

(54) **HANDSCHUHPAAR UND VERFAHREN ZUM AUSZEIHEN EINES HANDSCHUHPAARS**
PAIR OF GLOVES AND METHOD FOR REMOVING A PAIR OF GLOVES
PAIRE DE GANTS ET PROCÉDÉ POUR DÉCOLLER UNE PAIRE DE GANTS

(30) Priorität: 16.10.2003 AT 16312003
(43) Veröffentlichungstag der Anmeldung: 05.07.2006
(73) Patentinhaber: Schrödl, Berthold, 1040 Wien (AT)
(72) Erfinder: SCHRÖDL, Berthold, A-1040 Wien (AT)
(74) Vertreter: Sonn & Partner Patentanwälte
(86) Internationale Anmeldenummer: PCT/AT2004/000354
(87) Internationale Veröffentlichungsnummer: WO 2005/036997

(56) Entgegenhaltungen:
- AT-U- 6 608
- US-A- 4 876 747
- US-A- 5 467 483
- US-A- 5 579 539
- PATENT ABSTRACTS OF JAPAN Bd. 1996, Nr. 10, 31. Oktober 1996 (1996-10-31) -& JP 08 158122 A (MARUYAMA HAJIME), 18. Juni 1996 (1996-06-18)

## Beschreibung

Die Erfindung betrifft ein Handschuhpaar gemäß dem Oberbegriff des Anspruchs 1 sowie ein Verfahren zum Ausziehen eines solchen Handschuhpaars nach Anspruch 14.

In der US 4 876 747 A ist ein Handschuh beschrieben, der mit Hilfe eines Hakens ausgezogen werden kann, wobei die Handschuhe beider Hände bei Vorhandensein von zwei Haken auch gleichzeitig von den Händen des Benutzers abgezogen werden können. Dazu ist parallel zum Handschuhrand und auf der Handrückenseite der Stulpe eine Öffnung bzw.

Schleife angebracht, in welche ein an der Wand oder dgl. montierter Haken eingeführt werden kann. Darüber hinaus kann diese Schleife aber auch von der anderen Hand ergriffen und der Handschuh abgezogen werden, zum Ausziehen des zweiten Handschuhs ist dann aber wieder der Haken erforderlich.

Ein Handschuh aus Elastomermaterial ist aus der US 5 579 539 bekannt geworden, der daumenseitig am Stulpenteil oder am dem Handrückenteil gegenüberliegenden Teil der Stulpe, allenfalls auch am Handrückenteil selbst, jeweils vom hinteren Rand der Stulpe beabstandet, eine Ausziehhilfe aufweist. Damit ist bei entsprechender ergonomisch und anatomisch ungünstiger Verdrehung der Hände und/oder Unterarme ein mühsames gleichzeitiges Abstreifen beider Handschuhe möglich. Durch die notwendige Verdrehung der Hände ist aber die Gefahr des Abrutschens von der Ausziehhilfe sehr gross, wobei dann die verschmutzte Außenseite des Handschuhs auf die Haut des Trägers zurückschnellen kann.

Ebenfalls eine Ausziehhilfe, hier in Form von bei normaler Benutzung abgedeckten, am Handrückenteil der Stulpe angebrachten Streifen, Fäden, Laschen od. dgl., ist in der US 5 467 483 beschrieben. Mit dieser Ausziehhilfe ist jedoch ein gleichzeitiges Abstreifen beider Handschuhe kaum möglich. Da jedoch nicht mit ausreichender Sicherheit gewährleistet werden kann, dass die Ausziehhilfe nicht doch nach Öffnen der Abdeckung beschmutzt wird, ist ein Ergreifen der Ausziehhilfe mit einer nicht mehr geschützten Hand mit dem Risiko der Beschmutzung dieser Hand verbunden.

Es war nun die Aufgabe der vorliegenden Erfindung, einen Handschuh der eingangs angegebenen Art derart zu verbessern, dass in ergonomischer und einfacher Weise ein Ausziehen der Handschuhe ohne Hilfsmittel auch bei sehr kurzen Stulpen ermöglicht wird, ohne dass die verschmutzte oder kontaminierte Außenseite mit der Haut des Trägers in Kontakt kommt. Auch das Anziehen der Handschuhe soll gleichzeitig erleichtert und vereinfacht werden, ohne dass der Träger die Außenseite beispielsweise steriler Handschuhe mit nicht sterilen Körperteilen oder Hilfsmitteln berühren muss.

Eine Lösung der eingangs gestellten Aufgabe kann durch die Merkmale erzielt werden, dass eine Greifhilfe eines Handschuhs des Handschuhpaars an der den äußeren Knöchelbereich des Handgelenks bedeckenden Außenseite der Stulpe dieses Handschuhs angeordnet ist, sodass die Greifhilfe jedes Handschuhs in der Stellung der gegengleich parallel gehaltenen Arme und Hände von der jeweils anderen Hand ergriffen und gleichzeitig ausgezogen und gleichzeitig automatisch gewendet werden können, ohne dass die verschmutzte oder kontaminierte Außenseite der Handschuhe mit der Haut des Trägers in Kontakt kommt, und dass die Handschuhe des Handschuhpaars durch das weitere Ineinanderziehen miteinander verbunden werden. Dieser Winkelabstand betrifft das geometrische Zentrum der Greifhilfe, wohingegen der greifhilffreie Abstand zwischen zwei Greifhilfen allenfalls einen kleineren Winkelbereich einschließen kann, insbesondere bei sehr ausgedehnten Greifhilfen. Durch diese geometrische Anordnung der Greifhilfe kann die Greifhilfe jedes Handschuhs in einfacher Weise in der natürlichen Stellung der gegengleich parallel gehaltenen Arme und Hände von der jeweils anderen Hand ergriffen und zum Abziehen des Handschuhs genutzt werden. Die allenfalls verschmutzte oder kontaminierte Außenseite der Handschuhe hat dabei keinen Hautkontakt, sondern wird nur mit der durch den anderen Handschuh geschützten Hand berührt. Die Handschuhe werden beim gleichzeitigen und gegengleichen Ausziehen gleichzeitig automatisch gewendet, was die nachfolgende Handhabung vereinfacht und sicherer macht. Die Werte für den Winkelabstand der Greifhilfe gestatten für den überwiegenden Anteil von Handschuhträgern den ergonomisch günstigsten und auch am besten gegen unbeabsichtigtes Beschmutzen der Haut gesicherten Ausziehvorgang. Vorteilhafterweise ist gemäß einer Ausführungsform der Erfindung vorgesehen, dass die Greifhilfen der beiden Handschuhe im Wesentlichen diametral gegenüberliegend angeordnet sind.

Gemäß einer weiteren Ausführungsform des erfindungsgemäßen Handschuhpaars erstreckt sich zum leichteren und sicheren Ergreifen die zumindest eine Greifhilfe jedes Handschuhs über einen Winkelbereich von zumindest 90°.

Gemäß einer weiteren Ausführungsform kann die Greifhilfe ein- oder mehrmals unterbrochen sein, was das sichere Ergreifen der Greifhilfe auch bei beispielsweise feuchter Oberfläche erleichtert.

Einen ergonomisch besonders günstigen Ausziehvorgang zum gleichzeitigen Abstreifen beider Handschuhe gestattet eine Ausführungsform der Erfindung, bei welcher die Greifhilfe an der den äusseren Knöchelbereich des Handgelenks bedeckenden Außenseite der Stulpe näher dem Handteil des Handschuhs liegt als eine Greifhilfe an einem die Handinnenseite bedeckenden Umfangsabschnitt der Stulpe.

Eine bevorzugte Ausführungsform der Erfindung sieht.vor, dass die zumindest eine Greifhilfe jedes Handschuhs zumindest eine durchgehende Öffnung aufweist. Diese Art von Greifhilfe gestattet das Hinein- und Hindurchschlüpfen in die Greifhilfe mit einem oder mehreren Fingern der anderen Hand oder auch einem stab- oder hakenförmigen Hilfsmittel und bietet damit weitgehende Sicherheit gegen Abrutschen von bzw. aus der Greifhilfe.

Vorzugsweise ist die Greifhilfe jedes Handschuhs als sich von der Stulpe erhebende Schlaufe ausgebildet. Diese Art von Greifhilfe schliesst auch Varianten mit bei normaler Benützung des Handschuhs an der Stulpe flach anliegenden Lasche mit ein.

Eine andere vorteilhafte Variante sieht vor, dass zumindest eine Greifhilfe eine zumindest einseitig offene Tasche aufweist. Auch für derartige Taschen ist ein sicheres Ergreifen durch Hineinschlüpfen beispielsweise mit einem oder mehreren Fingern der anderen Hand oder einem Hilfswerkzeug gewährleistet.

Um das Hineinschlüpfen in die Lasche oder auch Tasche mit einem oder mehreren Fingern der anderen Hand zu erleichtern bzw. in ergonomisch günstiger Haltung zu gestatten, kann gemäß einem weiteren Merkmal der Erfindung vorgesehen sein, dass die Orientierung zumindest einer Greifhilfe um maximal 80° von der Längsachse der Stulpe abweicht, vorzugsweise im wesentlichen mit der Längsachse der Stulpe übereinstimmt.

Andererseits könnte aber auch zumindest eine Greifhilfe im wesentlichen quer zur Längsachse der Stulpe orientiert sein.

Das Ergreifen und auch das sichere Halten während der Handhabung des Handschuhs bei Aus- als auch beim Anziehen kann für viele Arten von Greifhilfen dadurch erleichtert werden, wenn zumindest eine Greifhilfe an einem Zwischenteil vorgesehen oder damit einstückig ausgeführt ist, welcher Zwischenteil selbst an der Stulpe angebracht oder damit einstückig ausgeführt ist.

Alternativ zu den oben genannten Ausführungen von Greifhilfen kann gemäß einer weiteren Ausführungsform der Erfindung zumindest eine Greifhilfe als lappen-, zungen- oder fadenförmiger Anhang ausgebildet sein. Allenfalls können am Ende oder im Verlauf derartige, speziell lappen- oder zungenförmiger Greifhilfen auch Öffnungen, Einschnitte od. dgl. vorgesehen sein, die ein Hinein- und Hindurchschlüpfen mit einem oder mehreren Fingern der anderen Hand oder einem Hilfswerkzeug gestatten.

Eine weitere alternative Ausführungsform der Erfindung ist dadurch gekennzeichnet, dass die zumindest eine Greifhilfe jedes Handschuhs als zur Innenseite des Handschuhs offene, hohle Ausbuchtung des Handschuhs ausgebildet ist. Auf diese Weise bildet sich beim auf der Hand befindlichen Handschuh eine Aufwölbung von dessen Material oder auch eine Falte, die dann mit der anderen Hand leicht ergriffen werden kann, im Gegensatz zu glatt anliegendem Material. Diese Greifhilfe kann im wesentlichen kreissymmetrisch oder auch länglich und allenfalls mit einer Vorzugsrichtung ausgebildet sein.

Vorteilhafterweise können gemäß einer weiteren Ausführungsform der Erfindung aneinander anschließend in Umfangsrichtung der Stulpe zumindest zwei Greifhilfen mit unterschiedlicher Orientierung zur Längsachse der Stulpe vorgesehen sein.

Auch bei einer einzelnen länglichen Greifhilfe kann mit dem gleichen Effekt vorgesehen sein, dass zumindest eine Greifhilfe zumindest einmal ihre Orientierung zur Längsachse der Stulpe ändert.

Vorteilhafterweise kann die zumindest eine Greifhilfe jedes Handschuhs mit die Griffigkeit erhöhenden Oberflächenstrukturen versehen sein. Dies kann durch entsprechendes Aufrauen des Materials der Stulpen-Oberfläche geschehen als auch durch verschiedene Oberflächenstrukturen wie etwa Noppen, Rippen od. dgl. Auch sind viele unterschiedliche Formen dieser Oberflächenstrukturen möglich, etwa halbkugelförmige, im wesentlichen kegelförmige oder auch pyramidenförmige Noppen.

Sowohl bei zentralsymmetrischen als auch bei länglichen Greifhilfen kann das Festhalten dadurch erleichtert werden, dass zumindest eine Greifhilfe einen von der Oberfläche der Stulpe zumindest teilweise größer werdenden Querschnitt aufweist. Die Greifhilfe ist bei derartiger Ausbildung beispielsweise im Querschnitt ähnlich dem griechischen Buchstaben Omega oder ähnelt einem auf dem Kopf stehenden Kegel oder im Querschnitt einem auf der Spitze stehenden Dreieck.

Wenn gemäß einer vorteilhaften Ausführungsform der Erfindung im Bereich zumindest einer Greifhilfe ein Dehnungsbereich der Stulpe vorgesehen ist, wird das erste Stadium des Abziehens des Handschuhs wesentlich erleichtert.

Bei separat gefertigten und anschließend mit der Stulpe des Handschuhs verbundenen Greifhilfen besteht auch zur Verbesserung der Griffigkeit der Greifhilfe die Möglichkeit, dass zumindest eine Greifhilfe aus anderem, vorzugsweise griffigerem Material als die Stulpe des Handschuhs angefertigt ist.

Alternativ oder auch zusätzlich zu in Umfangsrichtung - oder allenfalls auch in Längsrichtung des Handschuhs - aufeinanderfolgenden Greifhilfen können auch Anordnungen vorteilhaft sein, welche dadurch gekennzeichnet sind, dass aneinander anschließend und/oder einander überschneidend in Umfangsrichtung der Stulpe zumindest zwei Greifhilfen mit unterschiedlicher Orientierung zur Längsachse der Stulpe vorgesehen sind.

Die Festigkeit des Handschuhs und damit die Sicherheit gegen Beschädigung und unbeabsichtigtes Beschmutzen der Haut des Trägers durch zerstörte Bereiche des Handschuhs hindurch kann verbessert werden, indem die Greifhilfe und/oder zumindest der Übergangsbereich zwischen Stulpe und Greifhilfe verstärkt ist, beispielsweise mittels eingelagerter Verstärkungsfasern.

In der nachfolgenden Beschreibung soll die Erfindung anhand der beigefügten Zeichnungen von Ausführungsbeispielen erfindungsgemässer Handschuhe bzw. Handschuhpaare näher erläutert werden.

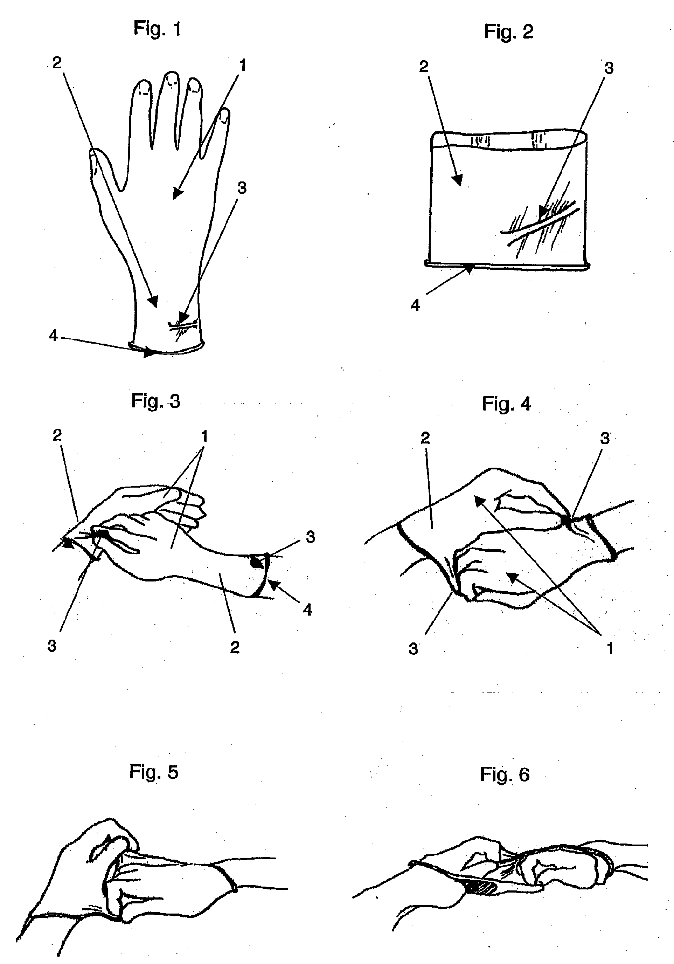

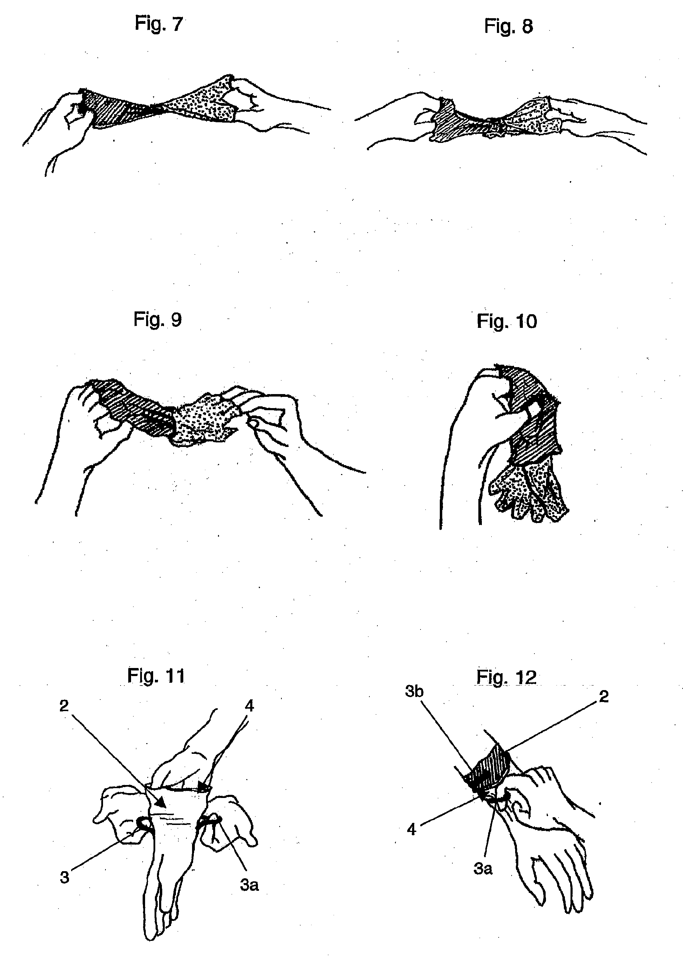

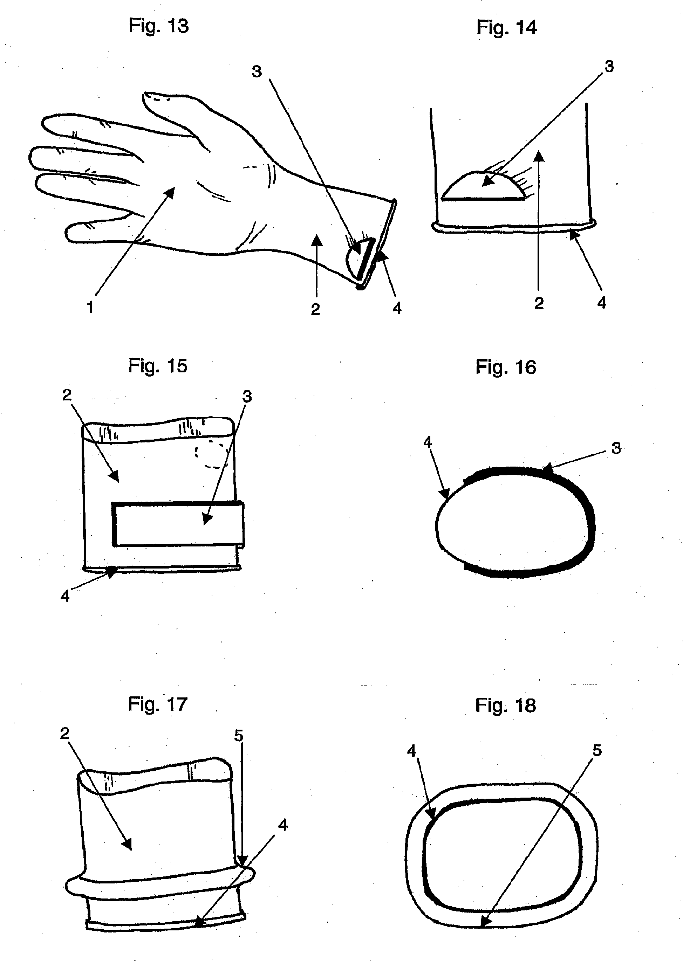

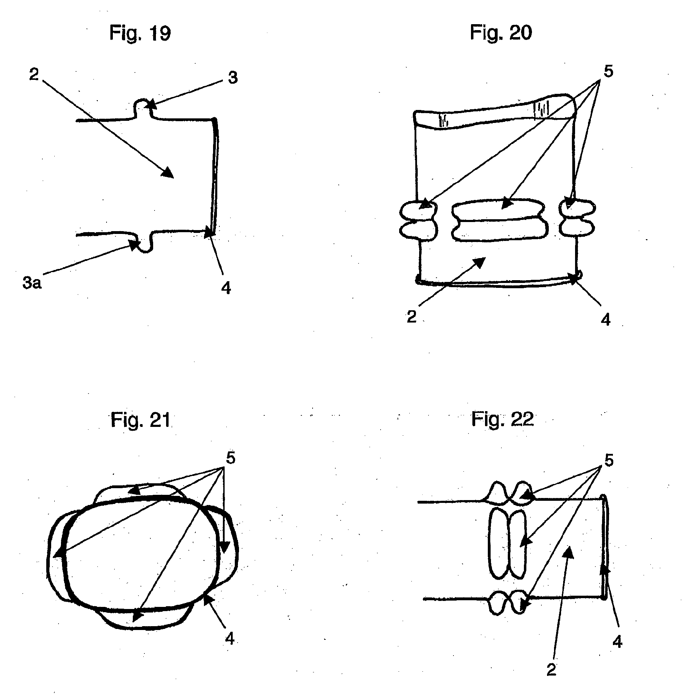

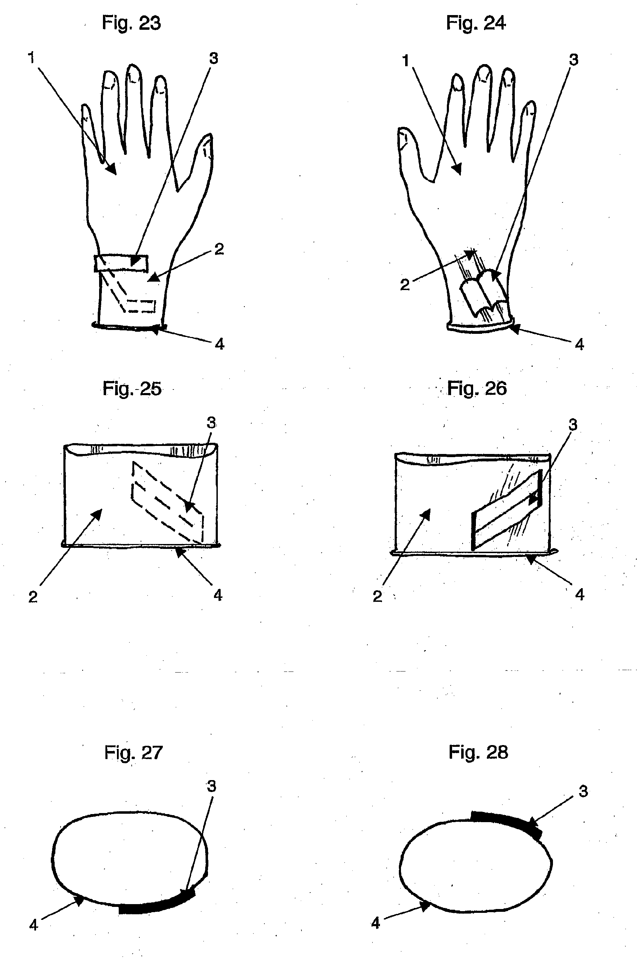

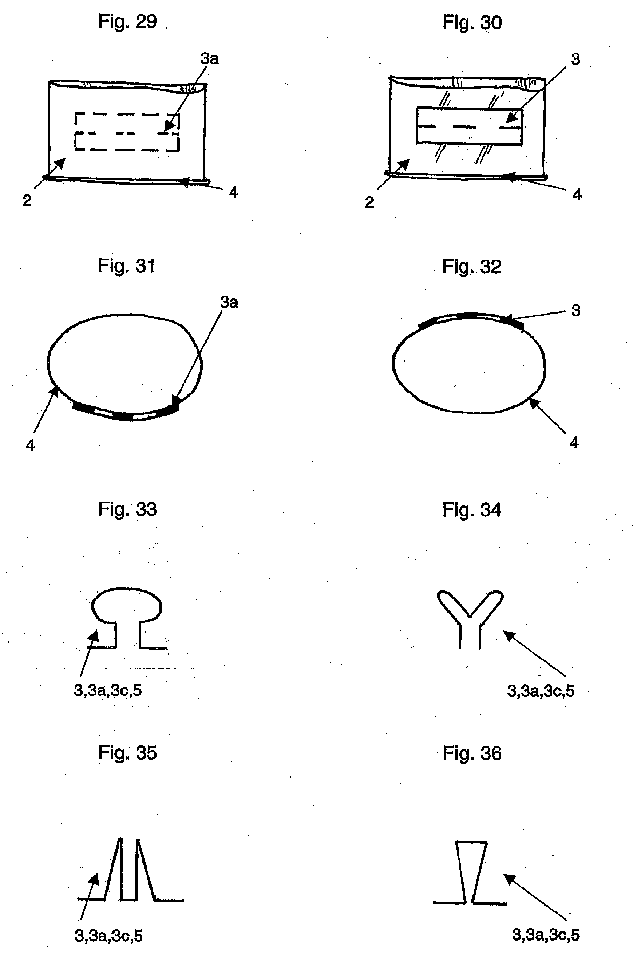

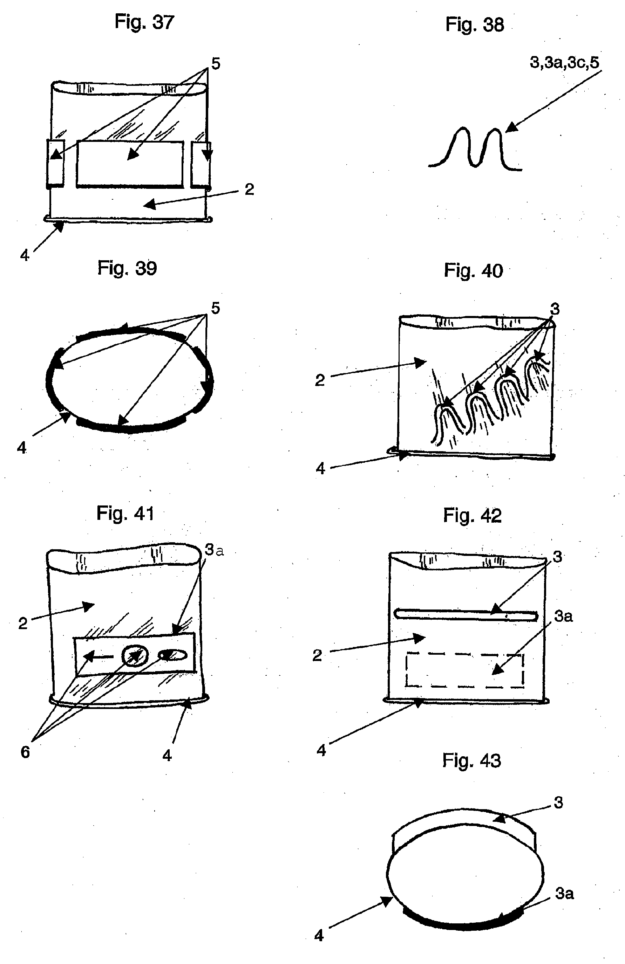

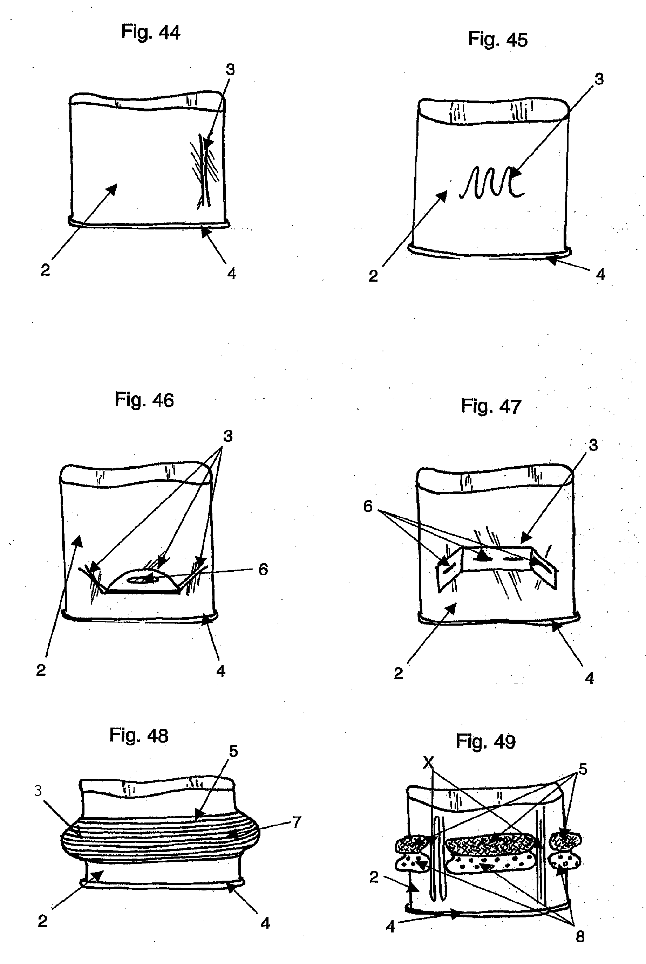

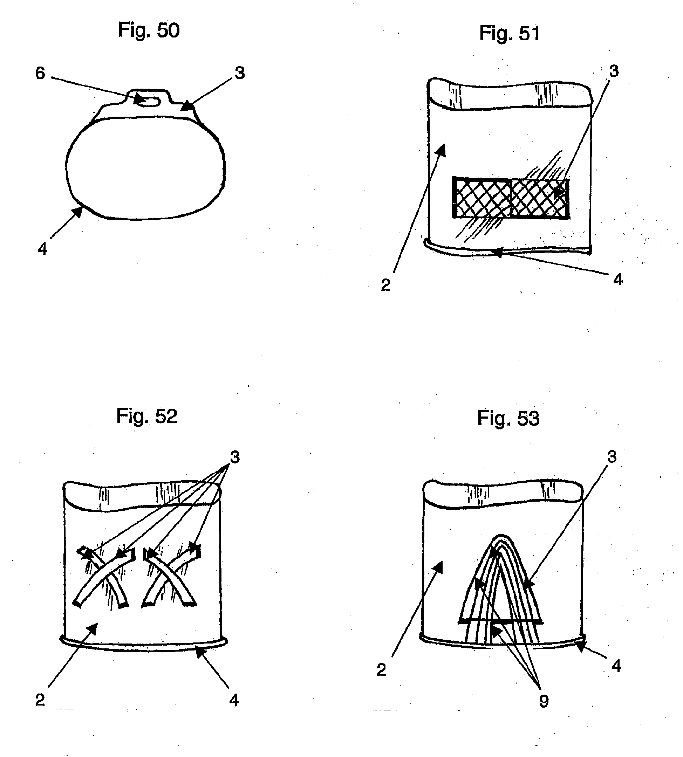

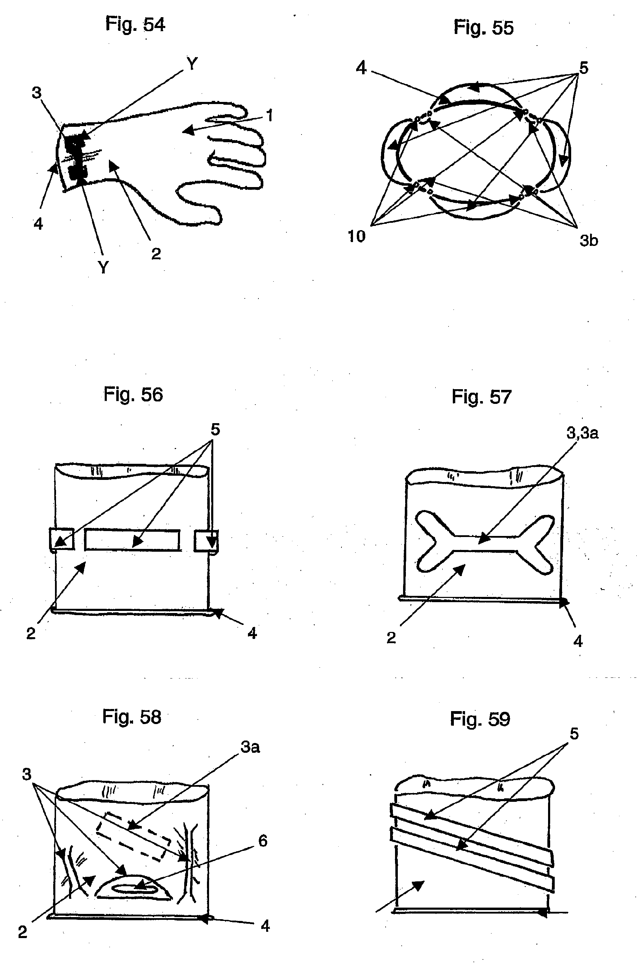

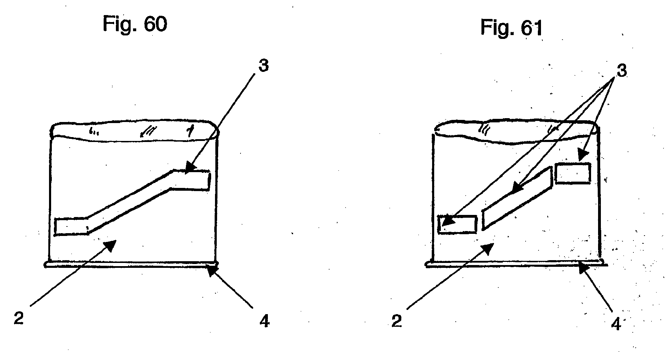

Dabei zeigt die Fig. 1 einen Handschuh mit einer einzigen laschenförmigen Greifhilfe, Fig. 2 ist eine Darstellung des Stulpenbereiches eines Handschuhs mit ebenfalls laschenförmiger Greifhilfe in vergrössertem Massstab, Fig. 3 bis 10 zeigen den gegengleichen Ausziehvorgang mit einem erfindungsgemässen Handschuh-Paar in perspektivischer Darstellung, Fig. 11 zeigt ein Stadium beim Anziehen eines erfindungsgemässen Handschuhs einer weiteren Ausführungsform mit zwei Greifhilfen, Fig. 12 zeigt ein Stadium beim Ausziehen eines erfindungsgemässen Handschuhs einer weiteren Ausführungsform mit zusätzlicher innenliegender Greifhilfe, Fig. 13 zeigt eine Greifhilfe in einer anderen Position an der Stulpe, Fig. 14 ist eine Darstellung der Stulpe der Fig. 13 in grösserem Massstab, Fig. 15 ist eine Darstellung einer sich über einen grösseren Umfang erstreckenden Greifhilfe, Fig. 16 ist eine Ansicht des Handschuhs der Fig. 15 von hinten, Fig. 17 zeigt eine Stulpe mit sich stegförmig über den gesamten Umfang erstreckender Greifhilfe, Fig. 18 ist eine Ansicht des Handschuhs der Fig. 17 von hinten, Fig. 19 zeigt eine Stulpe eines Handschuhs in Seitenansicht, mit zwei einander im wesentlichen diametral gegenüberliegenden Greifhilfen, Fig. 20 zeigt eine Stulpe mit Greifhilfen entlang des gesamten Umfangs, Fig. 21 ist eine Ansicht des Handschuhs der Fig. 20 von hinten, Fig. 22 zeigt eine Stulpe mit Greifhilfen ähnlich der Fig. 20 in Seitenansicht, Fig. 23 zeigt in der Draufsicht den linken Handschuh eines Handschuhpaares mit einer Greifhilfe, Fig. 24 zeigt einen rechten Handschuh, mit einer weiteren Variante einer Greifhilfe, Fig. 25 zeigt eine Stulpe eines linken Handschuhs eines Handschuhpaares in Draufsicht mit an der Unterseite angeordneter, gemäss einer weiteren Variante ausgeführten Greifhilfe, Fig. 26 zeigt die Stulpe des rechten Handschuhs des Handschuhpaares mit an der Knöchelseite oben angeordneter Greifhilfe, Fig. 27 und 28 zeigen die Stulpen der Fig. 25 und 26 in einer Ansicht von hinten, Fig. 29 und Fig. 30 zeigen jeweils eine Draufsicht auf Stulpen des linken bzw. rechten Handschuhs eines Handschuhpaares mit anders gestalteten Greifhilfen, Fig. 31 und 32 sind Ansichten der Handschuhe der Fig. 29 und 30 von hinten, Fig. 33 bis 36 zeigen unterschiedliche Arten von Greifhilfen in Seitenansicht, Fig. 37 zeigt eine umlaufende, jedoch unterbrochene Greifhilfe in Taschenform, Fig. 38 zeigt eine weitere Ausführungsform einer Greifhilfe in Seitenansicht, Fig. 39 ist eine Ansicht der Stulpe des Handschuhs der Fig. 37 von hinten, Fig. 40 zeigt einen Handschuh mit einer Reihe von nebeneinanderliegenden Greifhilfen, Fig. 41 ist eine Draufsicht auf eine Stulpe mit wieder anderer Ausführungsform der Greifhilfe, Fig. 42 zeigt eine Stulpe mit zwei Greifhilfen in unterschiedlicher Ausführungsform und diametral gegenüberliegend, Fig. 43 zeigt den Handschuh der Fig. 42 in Ansicht von hinten, Fig. 44 ist eine Ansicht einer Stulpe mit laschenförmiger Greifhilfe parallel zur Längsachse der Stulpe, Fig. 45 zeigt eine Stulpe mit fadenförmiger Greifhilfe, Fig. 46 ist eine Ansicht einer Stulpe mit umfangsmässig aufeinanderfolgenden drei Greifhilfen unterschiedlicher Ausgestaltung, Fig. 47 zeigt ebenfalls eine Stulpe mit einer Greifhilfe mit unterschiedlicher Ausrichtung an verschiedenen Umfangspositionen, Fig. 48 ist die Ansicht einer Stulpe mit umlaufender Greifhilfe mit die Griffigkeit erhöhender Oberflächenstruktur, Fig. 49 zeigt eine Stulpe mit Greifhilfen mit Oberflächenstrukturen zur Erhöhung der Griffsicherheit und mit Dehnungsbereichen zwischen den einzelnen Greifhilfen, Fig. 50 zeigt die Hinteransicht eines Handschuhs mit einer wieder anderen Ausführungsform einer Greifhilfe, Fig. 51 ist eine Draufsicht auf eine Stulpe mit anderer Ausführungsform der Greifhilfe, Fig. 52 zeigt eine Stulpe mit einander übergreifenden, unterschiedlich ausgerichteten Greifhilfen in Laschenform, Fig. 53 zeigt eine Greifhilfenvariante mit Verstärkungsfasern, Fig. 54 zeigt eine Ansicht einer Stulpe mit separat gefertigter und dann aufgebrachter, laschenförmiger Greifhilfe, die Fig. 55 und 56 sind eine Hinteransicht und eine Draufsicht auf eine Handschuhstulpe mit einer weiteren Ausführungsform einer umlaufenden Greifhilfe, Fig. 57 zeigt eine weitere Ausführungform einer Greifhilfe, Fig. 58 ist eine Darstellung einer Stulpe mit einer Gruppe von aneinander anschliessenden Greifhilfen, Fig. 59 zeigt eine weitere Ausführungsform für umlaufenden Greifhilfen und die Fig. 60 und 61 sind Darstellungen von Greifhilfen mit verschiedener Ausrichtung, einmal unmittelbar ineinander übergehend, dann mit kurzem Abstand aneinander anschliessend.

Die Fig. 1 zeigt in der Draufsicht von oben einen rechten Handschuh, bestehend aus einem einheitlichen Handschuhkörper H, wobei der Handschuhkörper einen eigentlichen Handteil 1 und eine daran anschliessende Stulpe 2 aufweist. Die Ausgestaltung des Handschuhs H findet hauptsächlich Verwendung bei dicht und unmittelbar an der Hand des Trägers anliegenden Varianten, vorzugsweise aus flüssigkeitsdichtem, elastischem Material, beispielsweise Gummi, welche vorzugsweise als Untersuchungs- oder Operationshandschuhe dienen. Nichtsdestotrotz erstrecken sich die Vorteile der Handschuhausführung auch auf alle anderen Handschuharten, wie auch Stoff-oder Lederhandschuhe oder auch alle Arten von Einweghandschuhen aus Plastik, wie in Supermärkten und an Tankstellen üblich.

An der Stulpe 2 ist nun außen an einem Umfangsabschnitt eine hier beispielsweise laschenförmige Greifhilfe 3a vorgesehen. Diese zumindest eine Greifhilfe 3a ist auf einem Umfangsteil der Stulpe 2 vorgesehen, welcher aussen den Knöchelbereich der Hand bedeckt, wobei diese Angabe aber lediglich als ungefähre Positionierung in Umfangsrichtung gesehen werden muss. Auch kann sich die Greifhilfe 3a über einen gewissen Winkelbereich im umfangsmässigen Knöchelbereich erstrecken. In Längsrichtung gesehen kann die Greifhilfe 3a an sehr unterschiedlichen Positionen vorgesehen sein, für Handschuhe mit sehr kurzen Stulpen 2 sogar in den Bereich des eigentlichen Handteils 1 wandern und im Bereich des Handgelenks oder sogar der Handkante liegen. Diese und alle weiteren noch zu erläuternden Greifhilfen sind vorteilhafterweise vom Handschuhkörper H so wenig entfernt bzw. stehen so wenig davon ab, dass sie einerseits leicht und sicher zu ergreifen sind, jedoch in keiner Weise bei Arbeits- und Untersuchungsvorgängen etc. stören. Auch sind sie derart weit vom hinteren Rand 5 der Stulpe 2 beabstandet, dass keine Gefahr besteht beim An- bzw. Ausziehvorgang mit der einen behandschuhten Hand das Gewebe (Haut) der gegenüberliegenden Hand zu berühren.

Fig. 2 zeigt die Greifhilfe 3 in grösserem Massstab, wobei erkennbar ist, dass diese Greifhilfe 3 einstückig mit der Handschuhstulpe 2 ausgeführt ist und sich derart über die der Haut des Trägers entgegengesetzte Oberfläche der Stulpe 2 erhebt, dass ein oder sogar mehrere Finger der anderen Hand oder auch ein Hilfsmittel zum Handhaben des Handschuhs hindurchgesteckt werden können. Bei diesem Hilfsmittel kann es sich etwa um Haken oder dgl. handeln, die fest angebracht sein oder mit der anderen Hand gehalten werden können.

Jede Art von Greifhilfe 3 kann in beliebiger sinnvoller Ausrichtung in bezug auf die Längsachse der Stulpe 2 vorgesehen sein. Die Fig. 2 zeigt eine schräge Anordnung beispielsweise einer laschenförmigen Greifhilfe ähnlich der Fig. 1, welche optimal die Ausübung einer Zugkraft vom Knöchelbereich des Handschuhs schräg über die Handrückenseite hin zur Daumenseite gestattet.

Fig. 3 zeigt den Beginn des Ausziehvorganges eines Paares erfindungsgemässer Handschuhe in perspektivischer Ansicht. Neben der bereits in Zusammenhang mit Fig. 1 und Fig. 2 beschriebenen Greifhilfe 3 im Knöchelbereich der Stulpe 2 ist vorzugweise am Handschuh H der anderen Hand eine weitere Greifhilfe 3 vorgesehen, die in Umfangsrichtung gesehen an einer dem Handrückenteil 4 gegenüberliegenden Position vorgesehen ist, oder an einer Stelle, die sich auf zumindest einer Seite daran anschliesst. Die erste Greifhilfe wie auch die weitere Greifhilfe 3 könnten auch als sich über etwa 180° erstreckende, vom Knöchelbereich auf die Handinnenseite reichende Strukturen ausgeführt sein.

Nach Ergreifen der Greifhilfe 3 am in der Fig. 3 linken Handschuh mit der anderen, d. h. rechten, Hand (Fig. 3) kann, wie in Fig. 4 dargestellt ist, die im Knöchelbereich an der Aussenseite des zweiten, rechten Handschuhs angeordnete Greifhilfe 3 von der linken Hand in einer ergonomisch günstigen Stellung beider Hände bzw. Unterarme zueinander ergriffen werden. Die Haut des Trägers kommt mit keiner verschmutzten oder kontaminierten äusseren Oberfläche der Handschuhe, in Kontakt. Beim weiteren Auseinanderziehen der Hände, wie es in den Fig. 5 und 6 zu sehen ist, werden dann gegengleich und im wesentlichen gleichzeitig die Handschuhe, von den Unterarmen und dann auch der Handfläche bzw. dem Handrücken abgezogen, wobei die Handschuhe auch bereits zum Teil mit ihrer normalerweise aussen liegenden Oberfläche in den jeweils anderen Handschuh hineingezogen werden. Beim weiteren Auseinanderziehen der Hände und dem Abziehen der Handschuhe auch noch von den Fingern beider Hände werden die Handschuhe schliesslich ganz gewendet und durch das weite Ineinanderziehen miteinander verbunden, wie in den Fig. 7 bis 10 zu erkennen ist. Der Benutzer kann daher, wie in Fig. 10 zu erkennen ist, die Handschuhe nach dem Ausziehen halten und handhaben, ohne mit deren allenfalls verschmutzter oder kontaminierter Aussenseite in Kontakt zu kommen.

Die erfindungsgemäss ausgestaltete und angeordnete Greifhilfe 3 erleichtert nicht nur wie oben erläutert den Ausziehvorgang, sondern der Benutzer kann den Handschuh selber leichter überstreifen, indem er ihn an einer Lasche od. dgl. ergreift und ihn durch Ziehen daran in Richtung Arm bzw. Körper überstreift. Das Gleiche kann auch, wie in Fig. 11 zu sehen ist, ein Helfer machen, der eventuell schon (sterile) Handschuhe trägt, wobei dann weitgehende Sterilität gewährleistet ist. Ausserdem kann diese zweite Person den Handschuh aufdehnen indem sie, wenn der Handschuh zwei oder mehrere Greifhilfen 3, 3a hat, diese auseinanderzieht und so die Öffnung des Handschuhs aufdehnt. Der Benützer des Handschuhs kann dann leichter in die aufgedehnte Handschuhöffnung (und den Handschuh) schlüpfen.

Wenn die Stulpe 2 des Handschuhs bereits in irgendeiner Weise ein Stück umgeschlagen ist, wenn allenfalls die aussen liegenden Greifhilfen 3, 3a verloren wurden oder durch die Finger gerutscht sind, kann der weitere Ausziehvorgang auch erleichtert werden, wenn zusätzlich zumindest eine Greifhilfe 3b innen in der Stulpe 2 vorgesehen ist, wie dies in Fig. 12 gezeigt ist. Vorzugsweise wird eine derartige Greifhilfe 3b innen in der Stulpe 2 näher dem Handteil 1 des Handschuhs liegen als eine Greifhilfe 3, 3a aussen auf der Stulpe 2.

Andere Arten von Greifhilfen 3, 3a,3b, deren Ausgestaltungen, Anordnungen und Orientierungen, sind dann beispielhaft aber nicht beschränkend in den Fig. 13 bis 54 dargestellt.

Neben Greifhilfen 3, 3a in der zuvor beschriebenen Ausführungsform als Lasche mit einer durchgehenden Öffnung können auch Greifhilfen 3, 3a in zungen- oder lappenförmiger Ausbildung vorgesehen sein, wie dies in Fig. 13 und 14 für eine an der Handinnenseite angeordnete Greifhilfe 3 dargestellt ist. Diese lappenförmige Greifhilfe 3 liegt normalerweise flach an der Oberfläche der Stulpe 2 auf, wie dies auch für die laschenförmigen und alle weiteren Ausführungsformen von Greifhilfen 3, 3a, 3b vorteilhafterweise vorgesehen ist.

In Fig. 15 ist in der Draufsicht eine über einen weiten Umfangsbereich umlaufende Greifhilfe 3 dargestellt, die sich um den eigentlichen Knöchelbereich noch auf die Handrückenseite und auch die Handunterseite der Stulpe 2 erstreckt, wie in der Ansicht von hinten in Fig. 16 deutlich zu erkennen ist. Die Greifhilfe 3 der Fig. 15 und 16 ist auch vom Aufbau her anders als die bisher erläuterten Greifhilfen 3, 3a, 3b, nämlich in Form einer nach vorne, d. h. zum Handteil 1 des Handschuhs geschlossenen und nach hinten hin offenen Tasche ausgeführt. Auch Ausführungen mit zentralem Steg und somit nach vorne als auch hinten offener Taschenform sind möglich, in welche die Finger der anderen Hand oder auch andere Hilfsmittel zum An- und Ausziehen von vorne und/oder von hinten hineingreifen und so die Greifhilfe sicher halten können.

Eine weitere Ausführungsform der Greifhilfe, welche nicht Gegenstand der Erfindung ist, ist in den Fig. 17 und 18 zu erkennen, wo eine den gesamten Umfang der Stulpe 2 umlaufende Erhebung 5 als Greifhilfe vorgesehen ist. Diese Erhebung 5 kann durch eine Ausbuchtung des Materials der Stulpe 2 selbst gebildet sein oder durch zusätzliches Material, das auf die Stulpe 2 aufgebracht ist. Für die Herstellung der Ausbuchtung - wie für jegliche Art von Greifhilfen in Form von Ausbuchtungen - kann die Tauchform für Handschuh mit einer entsprechenden Ausformung versehen sein. Diese Ausformung kann nach mittauchen und Fertigstellung des Handschuhs auf der Tauchform verbleiben, aus dem Handschuh entfernt werden oder auch allenfalls darin verbleiben. Die Tauchform kann auch eine permanente Ausbuchtung haben.

Selbstverständlich können für Handschuhpaare wie auch für Einzelhandschuhe die Stulpe 2 vollständig umrundende Greifhilfen 4 vorgesehen sein. Auch könnten diese umlaufende Greifhilfen 4 taschenförmig ausgebildet sein, sowohl mit nach hinten, d. h. zum hinteren Rand 5 der Stulpe 2 weisender, Öffnung als auch nach vorne offen oder abwechselnd in beide Richtungen offen.

Fig. 19 zeigt andere Greifhilfen 3, 3a in Ausbuchtungs-Ausführung, welche als im wesentlichen kreissymmetrische Erhebungen der Stulpe 2 ausgeführt und gegen das Handschuh-Innere hohl und offen sind. Letztere Ausführungsform kann auch für die umlaufende Erhebung 5 der Fig. 17 und 18 vorgesehen sein.

Eine den Fig. 17 und 18 ähnliche Ausführungsform der Greifhilfe ist in den Fig. 20 und 21 dargestellt. Zwei dicht in Richtung der Längsachse der Stulpe 2 nebeneinanderliegende und im wesentlichen senkrecht auf diese Achse orientierte Grate bilden eine mit Ausnahme von vier Unterbrechungen die Stulpe 2 komplett umrundende Greifhilfe 5. Die Grate der Greifhilfe 5 reichen im wesentlichen über einen Winkelbereich von ca. 45° und befinden sich - umfangsmässig gesprochen - auf der Handrückenseite, dem äusseren Knöchelbereich, der Daumenseite und der Handinnenseite der Stulpe 2.

Fig. 22 zeigt eine Stulpe 2 mit Greifhilfen 5 ähnlich der Fig. 20 in Seitenansicht, wieder als zwei nebeneinanderliegende und durch vier Unterbrechungen in vier getrennte Bereiche geteilte Grate ausgeführt. Während die Fig. 20 jedoch eine Ausführungsform darstellt, bei welcher die Greifhilfe 5 durch zusätzlich auf die Stulpe 2 aufgebrachtes Material gebildet ist, sind die Grate gemäss Fig. 22 durch Ausstülpungen der Stulpe 2 selbst, vorzugsweise nach innen hin offene, vorzugsweise während des Tauchvorganges bei der Herstellung ausgebildete Hohlräume, gebildet.

Die Greifhilfe 3 des linken Handschuhs eines Handschuhpaares verläuft auf der Handrückenseite des Knöchelbereiches im wesentlichen parallel zum hinteren Rand 4 der Stulpe 2, geht dann einen Abschnitt über, der schräg in Richtung dieses hinteren Randes 4 zur Handinnenseite verläuft und von dort wieder ein kleines Stück weitgehend parallel zum Rand 4 verläuft.

Die im oberen Knöchelbereich des rechten Handschuhs eines Handschuhpaares vorgesehene Greifhilfe 3 ist in Form von zwei Halbröhren ausgeführt, die gegenüber der Achse der Stulpe 2 schräg, in Richtung von der Knöchelseite auf die Daumenseite hin laufend, orientiert sind. Der Benutzer kann mit den Fingern der anderen Hand und/oder einem Hilfswerkzeug von beiden Seiten her in die halbröhrenförmige Greifhilfe hineinschlüpfen.

Fig. 25 zeigt eine Stulpe eines linken Handschuhs eines Handschuhpaares wie in den Fig. 23 und 24 in der Draufsicht. Als Greifhilfe 3 ist - durch strichlierte Darstellung angedeutet - an der Handinnenseite der Stulpe 2 und umfangsmässig im Daumenbereich, d. h. der Unterseite in Fig. 25, eine längliche, nach vorne und hinten hin offene Tasche angebracht.

Die Längsachse der als Greifhilfe 3 dienenden Tasche, und in gleicher Weise ihr zentraler, mit der Stulpe 2 verbundener Innensteg, ist gegenüber der Längsachse der Stulpe 2 geneigt, schliesst hier beispielsweise einen Winkel von ca. 40 mit dieser Längsachse der Stulpe 2 ein.

Die Greifhilfe 3 kann - in jeder ihrer Ausführungsvarianten - in einem derartigen Winkel zur Handschuhachse angeordnet sein, auch deutlich von einer normal auf die Längsachse orientierten Anordnung abweichend, dass zumindest ein Finger der gegenüberliegenden Hand ergonomisch, leicht und sicher die Greifhilfe erfassen und halten kann.

Fig. 26 zeigt die Stulpe des rechten Handschuhs des Handschuhpaares mit an der oberen Knöchelseite angeordneter Greifhilfe 3, die gleich der Greifhilfe 3 der Fig. 25 als beidseitig offene Tasche ausgeführt ist. Fig. 27 und 28 zeigen die Stulpen der Fig. 25 und 26 in einer Ansicht von hinten, in welcher die umfangsmässige Position der Greifhilfen als auch deren umfangsmässige Erstreckung zu erkennen ist.

Eine andere Ausführungsform und auch Anordnung von Greifhilfen 3 und 3a eines zusammengehörigen Handschuhpaares - ähnlich den Paaren der Fig. 23 bis 28 - zeigen die Fig. 29 und 30. Wieder wird durch strichlierte Darstellung der Greifhilfe 3 in Fig. 29 symbolisiert, dass sich diese auf der Unterseite, umfangsmässig der Handinnenseite der Stulpe 2, befindet, während die Greifhilfe 3a am gegenüberliegenden Handschuh der Fig. 30 auf der Handrückenseite der Stulpe 2 angebracht ist. Die Längsachse der Greifhilfen 3,3a sind hier nun genau senkrecht auf die Längsachse des Handschuhs orientiert und die Greifhilfen 3,3a erstrecken sich über einen umfangsmässigen Winkelbereich von ca. 90° der Stulpe 2. Wie besonders deutlich in den Ansichten von hinten der Fig. 31 und 32 zu erkennen ist, sind die Greifhilfen 3,3a beispielhaft durch vorne und hinten offene Taschen gebildet, bei welchen aber der zentrale Steg zwei Unterbrechungen aufweist, wodurch zwei Öffnungen gebildet sind, durch welche ein Finger der anderen Hand oder auch ein Hilfswerkzeug zum noch besseren Ergreifen der Greifhilfen 3,3a hindurchschlüpfen kann.

Dem besseren Ergreifen der Greifhilfen 3,3a können auch bestimmte Querschnittsformen dienen. So zeigt die Fig. 33 eine dem griechischen Buchstaben "Omega" ähnliche Form einer Greifhilfe, die allenfalls auch in eine pilzförmige Ausführung mit nach unten abgebogenem Aussenrand übergehen könnte. Auch die ähnliche Querschnittsverbreiterung nach oben hin der Greifhilfe der Fig. 34, in Form des Buchstaben "γ", erleichtert das sichere Halten der Greifhilfe. Eine andere Variante der Greifhilfe ist in Fig. 35 dargestellt, weiche im Querschnitt bzw. der Seitenansicht zwei Dreiecken entspricht, die eine einander zugewandte, parallele Seite aufweisen. Ein Greifhilfen-Querschnitt in Form abgerundeter, nebeneinander- liegender Grate ist in Fig. 38 gezeigt. Bevorzugt sind jedoch sich nach oben verbreiternde Formen, wie etwa Greifhilfen mit dem beispielsweise in Fig. 36 gezeigten Querschnitt bzw. Seitenansicht in Form eines kopfstehenden Dreiecks.

Für manche Anwendungszwecke könnten allenfalls auch nach vorne offene Taschen als Greifhilfe vorteilhaft sein, wie beispielsweise bei der in Fig. 37 und 39 dargestellten Ausführungsform des erfindungsgemässen Handschuhs. Wie die Ansicht der Stulpe 2 von hinten in der Fig. 39 deutlich macht, kann auch eine taschenförmige Greifhilfe 5 im wesentlichen die Stulpe 2 umfangsmässig vollständig umlaufend und nur durch kleine Unterbrechungen in separate Bereiche unterteilt ausgeführt sein. Die einzelnen taschenförmigen Bereiche erstrecken sich winkelmässig über etwas mehr als 90° , sind am Handrückenbereich, an der Handinnenseite, im Knöchelbereich und im Daumenbereich des Umfangs der Stulpe 2 positioniert und sind durch schmale greifhilfefreie Stulpenbereiche voneinander getrennt.

Wie in Fig. 40 zu erkennen ist, können beliebig viele einzelne Greifhilfen bzw. Greifhilfenabschnitte aneinander anschliessend, mit oder ohne Abstand zueinander, vorgesehen sein.

Beispielhaft ist dies in Fig. 40 anhand von vier in einer schrägen Linie im oberen Knöchelbereich der Stulpe 2 angeordneten Einzellasschen dargestellt, von welchen jede geringfügig von der benachbarten Lasche beabstandet ist und wobei jede Lasche selbst in Richtung vom Knöchelbereich auf die Daumenseite der Hand ausgerichtet ist, um in dieser Richtung optimal Zug ausüben zu können.

Fig. 41 ist eine Draufsicht auf eine Stulpe mit wieder anderer Ausführungsform der Greifhilfe 3a auf der Handrückenseite der Stulpe 2. Eine taschenförmige Greifhilfe 3a mit senkrechter Orientierung auf die Längsachse des Handschuhs ist zusätzlich mit Öffnungen 6 in jenem Bereich versehen, welcher die Oberseite der Tasche bildet. Diese Öffnungen 6 können beispielsweise in Form von Schlitzen (ganz links), im wesentlichen kreisförmigen (mittig) oder auch elliptischen Löchern (ganz rechts) vorliegen, wobei bei mit einer Vorzugsrichtung versehenen Öffnungen 6 diese' Vorzugsrichtung in prinzipiell beliebiger Orientierung relativ zur Längsachse der Greifhilfe 3a und/oder der Stulpe 2 des Handschuhs vorliegen kann.

Wie als weiteres Ausführungsbeispiel in Fig. 42 dargestellt, können an einem Handschuh natürlich auch zwei oder mehr verschiedene Ausführungen von Greifhilfen 3,3a kombiniert sein, ebenso wie verschiedene Positionen und Ausrichtungen der Greifhilfen relativ zueinander und/oder zur Längsachse des Handschuhs. Im Beispiel der Fig. 42 ist auf der Handrückenseite der Stulpe 2 eine gerade, senkrecht zur Längsachse des Handschuhs orientierte gratförmige Erhebung als Greifhilfe 3 und diametral gegenüberliegend auf der Handinnenseite eine taschenförmige Greifhilfe 3a, ebenfalls senkrecht auf die Längsachse der Stulpe 2 stehend, vorgesehen. Die umfangsmässige Position ist deutlich in Fig. 43 dargestellt.

Mehrere Greifhilfen 3,3a können selbstverständlich nicht nur auf unterschiedlichen Handschuhen, sondern auch auf ein und demselben Handschuh in Längsrichtung des Handschuhs unterschiedliche Positionen einnehmen. So ist im Ausführungsbeispiel der Fig. 42 die gratförmige obere Greifhilfe 3 weiter vom hinteren Rand 4 der Stulpe 2 beabstandet als die taschenförmige untere Greifhilfe 3a, die weiter vom Handteil 1 des Handschuhs entfernt liegt.

In jedem Fall ist aber auch die dem hinteren Rand 4 nächstliegende Greifhilfe 3, 3a oder 5 soweit vom hinteren Rand 4 der Stulpe 2 beabstandet, dass keinerlei Gefahr besteht beim Ergreifen der Greifhilfe 3,3a oder 5 mit einem verschmutzten oder kontaminierten Handschuh die nicht mehr von der Stulpe 2 bedeckte Haut oder Kleidung des Trägers zu berühren.

Fig. 44 ist eine Ansicht einer Stulpe 2 mit laschenförmiger Greifhilfe 3, welche parallel zur Längsachse der Stulpe 2 orientiert ist.

Eine ganz andere Ausführungsform einer Greifhilfe 3 zeigt die Fig. 45. Sie ist als im Ruhezustand schlangenförmig an der Stulpe 2 anliegender Faden ausgeführt, der mit der anderen Hand von der Stulpe 2 abgehoben und zum Ausüben eines Zuges in im wesentlichen beliebiger Richtung verwendet werden kann.

Mehrere, auch unterschiedlich ausgeführte, Greifhilfen können auch unmittelbar aneinander anschliessen, wie das in Fig. 46 beispielhaft dargestellt ist. Hier sind zu beiden Seiten einer zentralen, laschen- bzw. zungenförmigen Greifhilfe mit zusätzlich einem Langloch für sichereres Ergreifen laschenartig ausgebildete Greifhilfen angeordnet. Die unterschiedlichen, aneinander anschliessenden Greifhilfen 3 könnten gleich oder parallel orientiert sein, sie können aber auch wie dargestellt unterschiedliche Ausrichtung zueinander aufweisen.

Beispielhaft dafür zeigt die Fig. 46 eine Ausführungsform, bei welcher die äusseren Ende der aussenliegenden, laschenförmigen Bereiche der Greifhilfe 3 zum Handteil 1 des Handschuhs hin versetzt sind.

Fig. 47 zeigt eine Stulpe mit einer Greifhilfe 3 mit unterschiedlicher Ausrichtung an verschiedenen Umfangspositionen, wobei die verschiedenen Bereiche der Greifhilfe 3 jedoch im wesentlichen gleichartig ausgeführt sind, nämlich als taschenförmige Struktur mit Schlitzen in der oberen Taschenbegrenzung. Im Gegensatz zur Greifhilfe 3 der Fig. 46 sind hier aber die äusseren Ende der aussenliegenden Abschnitte der Greifhilfe 3 in Richtung auf den hinteren Rand 4 der Stulpe 2 hin versetzt.

Um das Ergreifen und das Halten der Greifhilfen während des An- oder Ausziehvorganges zu erleichtern, können verschiedene Massnahmen zur Erhöhung der Griffigkeit der Oberfläche im Bereich der Greifhilfen vorgesehen sein. So können, wie in Fig. 48 beispielhaft dargestellt ist, Rillen 7 vorhanden sein, welche bei länglichen Greifhilfen und auch im Fall von umlaufenden Greifhilfen 3 in Längsrichtung bzw. in Umfangsrichtung der Stulpe 2 verlaufen. Diese Rillen 7 können auch durch Falten des Materials der Stulpe 2 ausgebildet sein, was gleichzeitig die Aufweitung des Bereiches der Greifhilfe 3 gestattet, die damit noch leichter und einfacher gehalten werden kann.

Neben Rillen 7 wie in Fig. 48 sind auch anders strukturierte Oberflächenbereiche für die Greifhilfen 5 möglich. Neben einfach aufgerauhten Flächen können auch Noppen 8 oder andere Erhebungen flächig vorgesehen sein. Die Noppen 8 selbst können verschiedenste Ausbildungen aufweisen, wie beispielsweise im wesentlichen halbkreisförmig, zapfenförmig, kegel- oder pyramidenförmig, usw. Auch können unterschiedliche Ausbildungen in aneinandergrenzenden Bereichen vorgesehen sein, wie dies beispielhaft für die umlaufende, unterbrochene Greifhilfe 5 der Fig. 49 dargestellt ist. In Fig. 49 ist auch noch zusätzlich dargestellt, dass nicht nur die Greifhilfe 5 selbst (wie in Fig. 48) sondern dass auch die Zwischenbereiche X mit dehnungsfähiger Ausführung wie Rillen oder Falten oder dehnungsfähigem Material ausgestattet sein können.

Eine sich etwas über die Oberfläche der Stulpe 2, hier beispielhaft auf der Handrückenseite, erhebende Greifhilfe 3 ist Fig. 50 gezeigt. Sie besteht aus einem lappenförmigen Teil, dessen zentraler Bereich höher ausgeführt ist als die Seitenteile und der zusätzlich noch mit einem quer zur Stulpe 2 orientierten Langloch 6 versehen ist.

Fig. 51 zeigt in der Draufsicht eine weitere Ausführungsform einer Stulpe 2 mit einer taschenförmigen Greifhilfe 3, welche in einem Längsabschnitt nach hinten hin, auf den hinteren Rand 4 der Stulpe 2 hin, und im anschliessenden (in der Zeichnung rechten) Abschnitt nach vorne hin, auf den Handteil 1 des Handschuhs hin, offen ist. Zusätzlich ist zur Erhöhung der Griffigkeit die äussere Oberfläche der Greifhilfe 3 mit beispielsweise einer Waffelprägung versehen.

Dass die Greifhilfen 3, 3a, 3c, 5 gemäss der vorliegenden Erfindung nicht nur aneinander in Umfangsrichtung und auch in Längsrichtung der Stulpe 2 anschliessen können, sondern einander auch überschneiden können, ist beispielhaft in Fig. 52 dargestellt. Hier sind auf der Handrückenseite der Stulpe 2 vier laschenartige Greifhilfen 3 vorgesehen, von welchen jeweils zwei Laschen einander kreuzförmig überschneidend angeordnet sind. Die einzelnen Laschen sind beispielhaft um jeweils im wesentlichen 45° gegenüber der Längsachse der Stulpe 2 geneigt und ebenfalls beispielhaft sind jeweils zwei kreuzförmige Laschenpaare nebeneinander in im wesentlichen gleichem Abstand vom hinteren Rand 4 der Stulpe 2 positioniert.

Fig. 53 zeigt eine zungenförmige, auf den Handteil des Handschuhs hin weisende Greifhilfe 3 am Handrückenteil der Stulpe 2, wobei in die Greifhilfe 3 selbst und den Verbindungsbereich Verstärkungsfasern 9 eingelagert sind. Diese Art der Verstärkung des Verbindungs- bzw. Übergangsbereiches von Greifhilfe und Stulpe kann selbstverständlich für jede Ausführungsform von Greifhilfe und Stulpe vorgesehen sein.

Beispielhaft für separat gefertigte und dann auf die Stulpe 2 aufgebrachte Greifhilfen 3 ist in Fig. 54 eine laschenförmiger Greifhilfe 3 für die Handrückenseite des Handschuhs dargestellt, welche an ihren der Stulpe 2 zugewanden Enden plättchenartige Verbreiterungen aufweist, mit welchen die Greifhilfe 3 mit der Stulpe 2 vorzugsweise verklebt werden kann.

Eine umlaufende Greifhilfe 5 kann aber auch gemäss der in den Fig. 55 und 56 dargestellten Ausführungsform aufgebaut sein. Dabei ist ein geschlossenes oder mit beiden Enden am Handschuh befestigtes Band an mehreren Stellen, hier an acht Stellen 10, durch die Stulpe 2 des Handschuhs hindurchgeführt, vorzugsweise abgedichtet oder an den Durchführungsstellen mit dem Material der Stulpe dicht verbunden, beispielsweise verklebt oder verschweisst. Dadurch entstehen an der Aussenseite des Handschuhs mehrere, hier vier, Schlaufen als aussenliegende Greifhilfen 5 und gleichzeitig könnten die innenliegenden Abschnitte des Bandes, hier ebenfalls vier, bei ausreichendem Abstand zwischen den Durchgangspunkten durch die Stulpe 2, als innere Greifhilfe 3b genutzt werden.

Fig. 57 zeigt eine weitere Ausführungform einer Greifhilfe 3,3a, bei welcher nach aussen hin verzweigte Randabschnitte durch einen geraden, parallel zum hinteren Rand 4 der Stulpe 2 liegenden Abschnitt verbunden sind.

Auf der Stulpe 2 der Fig. 58 ist eine Gruppe von aneinander anschliessenden Greifhilfen 3, mit Abstand voneinander positioniert, auch beispielhaft mit unterschiedlicher Ausrichtung gegenüber der Längsachse der Stulpe 2 und in unterschiedlichen Ausführungsformen, d. h. zentral eine lappenförmige Greifhilfe 3 mit Langloch 6 und seitlich zwei laschenförmige Greifhilfen 3, dargestellt. Durch strichlierte Darstellung symbolisiert ist auch an der Handinnenseite der Stulpe 2 beispielhaft eine hier als zumindest einseitig offene Tasche ausgeführte Greifhilfe 3a vorgesehen.

Fig. 59 zeigt beispielhaft, dass auch umlaufende Greifhilfen 5 gruppenweise vorgesehen sein können. Weiters ist durch die Fig. 59 beispielhaft dargestellt, dass auch diese umlaufenden Greifhilfen 5 schräg gegenüber der Längsachse der Stulpe 2 orientiert sein können, allenfalls natürlich auch für jede der Greifhilfen 5 der Gruppe unterschiedlich.

Fig. 60 und 61 sind Darstellungen von Greifhilfen 3, die sich über einen grösseren Winkelbereich vom äusseren Knöchelbereich über den Handrückenbereich der Stulpe 2 erstrecken und in ihrem Verlauf beispielhaft zweimal die Ausrichtung gegenüber der Längsachse der Stulpe 2 ändern. Während die Greifhilfe 3 der Fig. 60 aus drei Abschnitten besteht, die unmittelbar ineinander übergehen, zeigt die Fig. 61 eigentlich eine Gruppe mit beispielhaft drei einzelnen, unterschiedlich ausgerichteten und mit kurzem Abstand aneinander anschliessenden Greifhilfen 3.

## Patentansprüche

1. Handschuhpaar, bestehend aus zwei Handschuhen, insbesonders Arbeits-, Untersuchungs- oder Operationshandschuhen, mit jeweils einem einheitlichen Handschuhkörper, vorzugsweise aus flüssigkeitsdichtem, elastischem Material, beispielsweise Gummi, wobei der Handschuhkörper einen eigentlichen Handteil (1) und eine daran anschließende Stulpe (2) aufweist, wobei an jedem Handschuh des Handschuhpaars an der äußeren Oberfläche der Stulpe (2) und beabstandet vom hinteren Rand (4) der Stulpe (2) an einem Umfangsabschnitt zumindest eine Greifhilfe (3, 3a, 5) vorgesehen ist, wobei die Greifhilfen (3, 3a, 5) auf beiden Handschuhen auf aneinander angrenzenden Umfangsabschnitten der Stulpe (2) vorgesehen sind und einen Winkelabstand von zumindest 135° aufweisen, **dadurch gekennzeichnet, dass** eine Greifhilfe (3, 5) eines Handschuhs des Handschuhpaars an der den äußeren Knöchelbereich des Handgelenks bedeckenden Außenseite der Stulpe (2) dieses Handschuhs angeordnet ist, sodass die Greifhilfe jedes Handschuhs in der Stellung der gegengleich parallel gehaltenen Arme und Hände von der jeweils anderen Hand ergriffen und gleichzeitig und gegengleich ausgezogen und gleichzeitig automatisch gewendet werden können, ohne dass die verschmutzte oder kontaminierte Außenseite der Handschuhe mit der Haut des Trägers in Kontakt kommt, und dass die Handschuhe des Handschuhpaars durch das weitere Ineinanderziehen miteinander verbunden werden.

2. Handschuhpaar nach Anspruch 1, **dadurch gekennzeichnet, dass** die Greifhilfen (3, 3a, 5) der beiden Handschuhe im Wesentlichen diametral gegenüberliegend angeordnet sind.

3. Handschuhpaar nach Anspruch 1 oder 2, **dadurch gekennzeichnet, dass** sich die zumindest eine Greifhilfe (3, 3a, 5) jedes Handschuhs über einen Winkelbereich von zumindest 90° erstreckt.

4. Handschuhpaar nach einem der Ansprüche 1 bis 3, **dadurch gekennzeichnet, dass** die Greifhilfe (3, 3a, 5) jedes Handschuhs ein- oder mehrmals unterbrochen ist.

5. Handschuhpaar nach einem der Ansprüche 1 bis 4, **dadurch gekennzeichnet, dass** die Greifhilfe (3, 5) an der den äußeren Knöchelbereich des Handgelenks bedeckenden Außenseite der Stulpe (2) näher dem Handteil (1) des Handschuhs liegt als eine Greifhilfe (3a, 5) an einem die Handinnenseite bedeckenden Umfangsabschnitt der Stulpe (2).

6. Handschuhpaar nach einem der vorhergehenden Ansprüche, **dadurch gekennzeichnet, dass** die zumindest eine Greifhilfe (3, 3a, 3b, 5) jedes Handschuhs zumindest eine durchgehende Öffnung (6) aufweist.

7. Handschuhpaar nach Anspruch 6, **dadurch gekennzeichnet, dass** die Greifhilfe (3, 3a) jedes Handschuhs als sich von der Stulpe (2) erhebende Schlaufe ausgebildet ist.

8. Handschuhpaar nach einem der vorhergehenden Ansprüche, **dadurch gekennzeichnet, dass** die zumindest eine Greifhilfe (3, 3a) jedes Handschuhs als zur Innenseite des Handschuhs offene, hohle Ausbuchtung des Handschuhs nach außen ausgebildet ist.

9. Handschuhpaar nach einem der vorhergehenden Ansprüche, **dadurch gekennzeichnet, dass** die zumindest eine Greifhilfe (3, 3a, 5) jedes Handschuhs mit die Griffigkeit erhöhenden Oberflächenstrukturen (8) versehen ist.

10. Handschuhpaar nach einem der vorhergehenden Ansprüche, **dadurch gekennzeichnet, dass** die zumindest eine Greifhilfe (3, 3a, 5) jedes Handschuhs einen von der Oberfläche der Stulpe (2) zumindest teilweise größer werdenden Querschnitt aufweist.

11. Handschuh bzw. Handschuhpaar nach einem der vorhergehenden Ansprüche, **dadurch gekennzeichnet, dass** im Bereich zumindest einer Greifhilfe (3, 3a, 5) jedes Handschuhs ein Dehnungsbereich (X) der Stulpe (2) vorgesehen ist.

12. Handschuhpaar nach einem der vorhergehenden Ansprüche, **dadurch gekennzeichnet, dass** die zumindest eine Greifhilfe (3, 3a, 5) jedes Handschuhs aus anderem, vorzugsweise griffigerem Material als die Stulpe (2) des Handschuhs angefertigt oder damit versehen ist.

13. Handschuhpaar nach einem der vorhergehenden Ansprüche, **dadurch gekennzeichnet, dass** die Greifhilfe (3, 3a, 5) und/oder zumindest der Übergangsbereich zwischen Stulpe (2) und Greifhilfe (3, 3a, 5) verstärkt ist, beispielsweise mittels eingelagerter Verstärkungsfasern (9).

14. Verfahren zum Ausziehen eines Handschuhpaars nach einem der Ansprüche 1 bis 13, **dadurch gekennzeichnet, dass** mit der einen Hand des Trägers eine Greifhilfe (3, 5) des Handschuhs der anderen Hand ergriffen wird, welche an der Außenseite der Stulpe liegt, die den äußeren Knöchelbereich des Handgelenks der anderen Hand bedeckt, und in der Stellung der gegengleich parallel gehaltenen Arme und Hände des Trägers des Handschuhpaars jeweils eine Greifhilfe (3, 5) eines Handschuhs von der jeweils anderen Hand ergriffen wird, und die Handschuhe gleichzeitig und gegengleich ausgezogen und während des Ausziehvorgang automatisch gewendet werden, ohne dass die verschmutzte oder kontaminierte Außenseite der Handschuhe mit der Haut des Trägers der Handschuhe in Kontakt kommt, und dass die Handschuhe des Handschuhpaars schließlich ganz gewendet und durch das weitere Ineinanderziehen miteinander verbunden werden.

15. Verfahren nach Anspruch 14, **dadurch gekennzeichnet, dass** mit der anderen Hand eine Greifhilfe (3, 5) des Handschuhs der einen Hand ergriffen wird, welche an einem die Handinnenseite der einen Hand des Trägers bedeckenden Umfangsabschnitt der Stulpe (2) des Handschuhs liegt.

## Claims

1. A pair of gloves, consisting of two gloves, in particular work, examining or operating gloves, each having a unified glove body, preferably made from liquid-tight, elastic material, for example, rubber, wherein the glove body has an actual hand part (1) and an adjoining cuff (2), wherein at least one gripping aid (3, 3a, 5) is provided on each glove of the pair of gloves, on the outer surface of the cuff (2) and at a distance from the rear edge (4) of the cuff (2), wherein the gripping aids (3, 3a, 5) are provided on both gloves on adjacent circumferential sections of the cuff (2) and have an angular distance of at least 135°, **characterized in that** a gripping aid (3, 5) of one glove of the pair of gloves is arranged on the outer side of the cuff (2) of this glove covering the outer wrist bone region of the wrist so that in the position of the arms and hands held in a mirror-inverted fashion in parallel, the gripping aid of each glove can be grasped by the respectively other hand and can be removed simultaneously and in a mirror-inverted fashion and at the same time can be automatically turned inside out without the polluted or contaminated outer side of the glove coming in contact with the skin of the wearer and the gloves of the pair of gloves can be joined together by further drawing into each other.

2. The pair of gloves according to claim 1, **characterized in that** the gripping aids (3, 3a, 5) of the two gloves are arranged substantially diametrically opposite one another.

3. The pair of gloves according to claim 1 or 2, **characterized in that** the at least one gripping aid (3, 3a, 5) of each glove extends over an angular region of at least 90°.

4. The pair of gloves according to one of claims 1 to 3, **characterized in that** the gripping aid (3, 3a, 5) of each glove is interrupted once or several times.

5. The pair of gloves according to any one of claims 1 to 4, **characterized in that** the gripping aid (3, 5) on the outer side of the cuff (2) covering the outer wrist bone region of the wrist lies closer to the hand part (1) of the glove than a gripping aid (3a, 5) on a circumferential section of the cuff (2) covering the hand inner side.

6. The pair of gloves according to any one of the preceding claims, **characterized in that** the at least one gripping aid (3, 3a, 3b, 5) of each glove has at least one continuous opening (6).

7. The pair of gloves according to claim 6, **characterized in that** the gripping aid (3, 3a) of each glove is configured as a loop raised from the cuff (2).

8. The pair of gloves according to any one of the preceding claims, **characterized in that** the at least one gripping aid (3, 3a) of each glove is configured on the outside as a hollow bulge of the glove which is open towards the inside of the glove.

9. The pair of gloves according to any one of the preceding claims, **characterized in that** the at least one gripping aid (3, 3a, 5) of each glove is provided with surface structures (8) which enhance the grip.

10. The pair of gloves according to any one of the preceding claims, **characterized in that** the at least one gripping aid (3, 3a, 5) of each glove) has a cross-section which becomes larger from the surface of the cuff (2) at least in parts.

11. The glove or pair of gloves according to any one of the preceding claims, **characterized in that** a stretch region (X) of the cuff (2) is provided in the area of at least one gripping aid (3, 3a, 5) of each glove.

12. The pair of gloves according to any one of the preceding claims, **characterized in that** the at least one gripping aid (3, 3a, 5) of each glove is made from or provided with a different material than the cuff (2) of the glove, preferably one with a better grip.

13. The pair of gloves according to any one of the preceding claims, **characterized in that** the gripping aid (3, 3a, 5) and/or at least the transition region between cuff (2) and gripping aid (3, 3a, 5) is reinforced, for example by means of interstitial reinforcement fibres (9).

14. A method for removing a pair of gloves according to any one of claims 1 to 13, **characterized in that** with the one hand of the wearer, a gripping aid (3, 5) of the glove of the other hand is grasped which lies on the outer side of the cuff, which covers the outer wrist bone region of the wrist of the other hand and in the position of the arms and hands of the wearer of the pair of gloves held parallel in mirror-inverted manner, respectively one gripping aid (3, 5) of one glove is grasped by the respectively other hand and the glove is at the same time and in mirror-inverted manner withdrawn and automatically turned inside out during the removing process without the polluted or contaminated outer side of the glove coming in contact with the skin of the wearer of the glove and that the glove of the pair of gloves is finally turned completely and joined together by the further drawing into each other.

15. The method according to claim 14, **characterized in that** with the other hand, a gripping aid (3, 5) of the glove of the one hand is grasped, which lies on a circumferential section of the cuff (2) of the glove covering the inner side of the hand of the wearer.

## Revendications

1. Paire de gants, constituée de deux gants, en particulier des gants de travail, médicaux ou chirurgicaux, comportant chacun un corps de gant uniforme, de préférence dans un matériau élastique, étanche aux liquides, tel que le caoutchouc, le corps de gant comportant une partie formant la main (1) proprement dite et une manchette (2) prolongeant cette dernière, au moins un moyen de préhension (3, 3a, 5) étant prévu sur chaque gant de la paire de gants sur la surface extérieure de la manchette (2) et à distance du bord arrière (4) de la manchette (2) sur une partie périphérique, lesdits moyens de préhension (3, 3a, 5) étant prévus sur les deux gants sur des parties périphériques adjacentes de la manchette (2) et ayant une distance angulaire d'au moins 135°, **caractérisée en ce qu'**un moyen de préhension (3, 5) d'un gant de la paire de gants est disposé sur le côté extérieur, recouvrant la saillie osseuse extérieure du poignet, de la manchette (2) de ce gant, de telle sorte que, lorsque l'utilisateur maintient ses bras et ses mains dans une position parallèle inversée, le moyen de préhension de chaque gant peut être saisi respectivement par l'autre main et les gants peuvent être retirés simultanément et de manière inversée et peuvent être retournés simultanément automatiquement sans que la face extérieure sale ou contaminée du gant vienne en contact avec la peau de l'utilisateur, et **en ce que** les gants de la paire de gants peuvent être reliés entre eux en continuant à les tirer l'un dans l'autre.

2. Paire de gants selon la revendication 1, **caractérisée en ce que** les moyens de préhension (3, 3a, 5) des deux gants sont disposés de manière sensiblement diamétralement opposée.

3. Paire de gants selon la revendication 1 ou 2, **caractérisée en ce que** ledit au moins un moyen de préhension (3, 3a, 5) de chaque gant s'étend sur une zone angulaire d'au moins 90°.

4. Paire de gants selon l'une quelconque des revendications 1 à 3, **caractérisée en ce que** le moyen de préhension (3, 3a, 5) de chaque gant est interrompu une ou plusieurs fois.

5. Paire de gants selon l'une quelconque des revendications 1 à 4, **caractérisée en ce que** le moyen de préhension (3, 5) sur le côté extérieur de la manchette (2), lequel recouvre la saillie osseuse extérieure du poignet, est situé plus près de la partie formant la main (1) du gant qu'un moyen de préhension (3a, 5) sur une partie périphérique de la manchette (2), recouvrant la paume.

6. Paire de gants selon l'une quelconque des revendications précédentes, **caractérisée en ce que** ledit au moins un moyen de préhension (3, 3a, 3b, 5) de chaque gant comporte au moins une ouverture (6) débouchante.

7. Paire de gants selon la revendication 6, **caractérisée en ce que** le moyen de préhension (3, 3a) de chaque gant est réalisé sous la forme d'une boucle en relief sur la manchette (2).

8. Paire de gants selon l'une quelconque des revendications précédentes, **caractérisée en ce que** ledit au moins un moyen de préhension (3, 3a) de chaque gant est réalisé vers l'extérieur sous la forme d'un bombement creux, ouvert vers la face intérieure du gant.

9. Paire de gants selon l'une quelconque des revendications précédentes, **caractérisée en ce que** ledit au moins un moyen de préhension (3, 3a, 5) de chaque gant est munie de structures de surface (8) augmentant la maniabilité.

10. Paire de gants selon l'une quelconque des revendications précédentes, **caractérisée en ce que** ledit au moins un moyen de préhension (3, 3a, 5) de chaque gant comporte une section transversale s'agrandissant au moins partiellement en partant de la surface de la manchette (2).

11. Gant ou paire de gants selon l'une quelconque des revendications précédentes, **caractérisé en ce qu'**une zone de dilatation (X) de la manchette (2) est prévue dans la zone d'au moins un moyen de préhension (3, 3a, 5) de chaque gant.

12. Paire de gants selon l'une quelconque des revendications précédentes, **caractérisée en ce que** ledit au moins un moyen de préhension (3, 3a, 5) de chaque gant est réalisé dans un matériau différent de celui de la manchette (2) du gant, de préférence un matériau plus facilement préhensible ou est revêtu d'un tel matériau.

13. Paire de gants selon l'une quelconque des revendications précédentes, **caractérisée en ce que** le moyen de préhension (3, 3a, 5) et/ou au moins la zone de transition entre la manchette (2) et le moyen de préhension (3, 3a, 5) sont renforcés, par exemple au moyen de fibres de renfort (9) insérées.

14. Procédé permettant de retirer une paire de gants selon l'une des revendications 1 à 13, **caractérisé en ce que** l'utilisateur saisira avec une main un moyen de préhension (3, 5) du gant de l'autre main, lequel est situé sur la face extérieure de la manchette, laquelle couvre la saillie osseuse extérieure du poignet de l'autre main, et, lorsque l'utilisateur de la paire de gants, maintient ses bras et ses mains dans une position parallèle inversée, respectivement un moyen de préhension (3, 5) d'un gant sera saisi respectivement par l'autre main, et les gants seront retirés simultanément et de manière inversée et seront retournés simultanément automatiquement pendant le processus de retrait des gants, sans que la face extérieure sale ou contaminée du gant vienne en contact avec la peau de l'utilisateur des gants, et **en ce que** les gants de la paire de gants sont finalement complètement retournés et sont reliés entre eux par le fait que l'utilisateur continue à les tirer l'un dans l'autre.

15. Procédé selon la revendication 14, **caractérisé en ce qu'**avec l'autre main sera saisi un moyen de préhension (3, 5) du gant d'une main, lequel est situé sur la partie périphérique, recouvrant la paume de l'une des mains de l'utilisateur, de la manchette (2) du gant.
